(19) Europäisches Patentamt
European Patent Office
Office européen des brevets

(11) Veröffentlichungsnummer: **0 458 099 A2**

## (12) EUROPÄISCHE PATENTANMELDUNG

(21) Anmeldenummer: 91107099.3

(22) Anmeldetag: 02.05.91

(51) Int. Cl.⁵: C07C 309/17

(30) Priorität: 25.05.90 DE 4016863

(43) Veröffentlichungstag der Anmeldung:
27.11.91 Patentblatt 91/48

(84) Benannte Vertragsstaaten:
CH DE FR GB IT LI

(71) Anmelder: Chemische Fabrik Pfersee GmbH
Rehlinger Str. 1 Postfach 1153
W-8901 Langweid am Lech(DE)

(72) Erfinder: Sandner, Bernhard
Ortlerweg 12
W-8901 Diedorf(DE)
Erfinder: Demharter, Waltraud
Postweg 12
W-8901 Eppishofen(DE)
Erfinder: Koreny, Andrea
Brahmsstrasse 15
W-8900 Augsburg(DE)

(54) **Verfahren zur Herstellung von Sulfobernsteinsäuredialkylestersalzen.**

(57) Es wird ein Verfahren zur Herstellung von Sulfobernsteinsäuredialkylestersalzen in wäßriger Dispersion oder Lösung beschrieben.

Bei dem Verfahren wird ein Maleinsäure- oder Fumarsäuredialkylester mit einem Alkalimetallsulfit in wäßriger Dispersion unter Verwendung einer bestimmten Menge des als Umsetzungsprodukt entstehenden Salzes als Dispergator umgesetzt. Die Umsetzung erfolgt in Gegenwart eines Alkalimetallhydroxids bei einer Temperatur von 120 bis 150° C.

Das Verfahren bietet den Vorteil einer homogenen Reaktionsführung bei kurzen Reaktionszeiten. Durch Umsetzung in einem geschlossenen Gefäß wird $SO_2$-Emission unterbunden. Die entstehenden Salze können als Emulgatoren oder Netzmittel eingesetzt werden.

EP 0 458 099 A2

Die Erfindung bezieht sich auf ein Verfahren zur Herstellung von Sulfobernsteinsäuredialkylestersalzen in wäßriger Dispersion oder Lösung durch Erhitzen einer wäßrigen Dispersion, welche

a) Maleinsäuredialkylester und/oder Fumarsäuredialkylester

b) ein Sulfit eines Alkalimetalls und

c) das Sulfobernsteinsäuredialkylestersalz, welches durch Addition des Sulfits b) an den Diester a) entsteht, als Dispergator

enthält, in der die Komponenten a) und b) in solchen Mengen enthalten sind, daß pro C = C-Doppelbindung der Komponente a) 1,0 bis 1,1 Schwefelatome der Komponente b) vorliegen, und in der die Komponente c) in einer Menge von 1 bis 10 Gew.%, bezogen auf Gesamtdispersion, enthalten ist,

wobei man diese Dispersion so lange erhitzt, bis 95 bis 100 % der C = C-Doppelbindungen umgesetzt sind.

Ein solches Verfahren ist aus der DE-AS 25 46 847 bekannt.

Sulfobernsteinsäuredialkylestersalze sind als solche bekannt. Sie finden wegen ihrer oberflächenaktiven Eigenschaften unter anderem Verwendung als Emulgatoren und Netzmittel. Ein bekanntes Verfahren zu ihrer Herstellung besteht in der Addition eines Metallhydrogensulfits an die C = C-Doppelbindung von Malein- oder Fumarsäuredialkylester, wobei die entsprechenden sulfonsauren Metallsalze entstehen. Die Metalle sind hierbei in der Regel Alkalimetalle. An Stelle von Alkalimetallhydrogensulfiten können auch Pyrosulfite der Formel $M_2S_2O_5$ verwendet werden (M = Alkalimetall). Deren Umsetzung mit Maleinsäure- oder Fumarsäuredialkylestern in wäßrigem Medium führt ebenfalls zur Addition je einer $HSO_3^{\ominus}$-Gruppe an je eine C = C-Doppelbindung.

Verfahren der genannten Art sind beispielsweise beschrieben in US-PS 2 176 423, DD-PS 75 075 und GB-PS 760 121. Diese Verfahren weisen jedoch den Nachteil auf, daß die Addition des Sulfits an den Malein- oder Fumarsäurediester nicht in Gegenwart eines Dispergier- bzw. Emulgiermittels durchgeführt wird. Eine Reihe von Diestern der Malein- bzw. Fumarsäure, welche nach Sulfonierung zu in technischer Hinsicht interessanten Sulfobernsteinsäurediestersalzen führen, ist jedoch nicht wasserlöslich oder in Wasser selbst-dispergierend. Bei der Umsetzung solcher Diester mit Sulfit laufen die Verfahren der genannten Druckschriften nicht in homogener flüssiger Phase ab. Dies führt unter anderem zu dem Nachteil, daß selbst bei Umsetzung unter erhöhtem Druck die Geschwindigkeit der Sulfitaddition niedrig ist, wodurch verhältnismäßig lange Reaktionszeiten in Kauf genommen werden müssen.

Die DE-AS 19 12 531 beschreibt ein Verfahren, bei dem einem Maleinsäuredialkylester schrittweise eine wäßrige Sulfitlösung zugeführt wird. Hierbei wird als Homogenisierungsmittel das gewünschte Endprodukt (Sulfobernsteinsäurediester) zugesetzt, jedoch enthält die Maleinsäurediesterphase kein oder nur wenig Wasser. Dieses Verfahren besitzt ebenfalls unter anderem den Nachteil, daß es langwierig ist, bedingt durch die schrittweise langsame Zugabe von Sulfit.

Ein verbessertes Verfahren beschreibt die DE-AS 25 46 847. Hierbei wird in einer Eintopfreaktion in wäßrigem Medium die Addition von Sulfit oder Pyrosulfit an Maleinsäure- oder Fumarsäuredialkylester durchgeführt. Um die Reaktion in homogener, flüssiger Phase ablaufen zu lassen, kann das gewünschte Endprodukt (Sulfobernsteinsäurediestersalz) als Dispergator zugefügt werden. Dieses Verfahren ist jedoch noch mit dem Nachteil behaftet, daß hierbei unter Normaldruck unter Rückfluß gearbeitet wird. Hierdurch sind einerseits der Reaktionstemperatur und damit der Reaktionsgeschwindigkeit Grenzen gesetzt. Andererseits besteht, da in saurem Medium bei erhöhter Temperatur gearbeitet wird, die Gefahr der Entwicklung von Schwefeldioxid, so daß Maßnahmen zu treffen sind, damit kein Schwefeldioxid in die Umgebung entweicht.

Aufgabe der vorliegenden Erfindung war es, ein Verfahren zur Herstellung von wäßrigen Lösungen oder Dispersionen von Sulfobernsteinsäuredialkylestersalzen zu entwickeln, bei dem die Nachteile bekannter Verfahren vermieden werden, bei dem insbesondere eine hohe Reaktionsgeschwindigkeit erreicht wird. Die Aufgabe wurde gelöst durch ein Verfahren gemäß Oberbegriff von Anspruch 1, das dadurch gekennzeichnet ist, daß das Erhitzen der Dispersion in einem geschlossenen Gefäß unter dem Eigendruck des Systems bei einer Temperatur im Bereich von 120 bis 150 °C durchgeführt wird und daß der Dispersion vor dem Erhitzen ein Alkalimetallhydroxid in einer Menge von 0,1 bis 4 Gew.%, bezogen auf Gesamtdispersion, hinzugefügt wird.

Das erfindungsgemäße Verfahren weist folgende Vorteile auf:

Die Addition des Sulfits an die C = C-Doppelbindung findet in homogener wäßriger Phase statt, wodurch eine Erhöhung der Reaktionsgeschwindigkeit gegenüber einer Addition in heterogener Reaktion erzielt wird. Die Reaktionstemperatur von 120 bis 150 °C bewirkt eine weitere Steigerung der Reaktionsgeschwindigkeit gegenüber Verfahren, bei denen unter Normaldruck eine wäßrige Dispersion unter Rückfluß gekocht wird. Es wird keine schrittweise Zugabe von Sulfit zum Diester durchgeführt, sondern die gesamte Menge Sulfit wird vorgelegt, so daß die Umsetzung in einer "Eintopfreaktion" durchgeführt wird. Das Arbeiten im geschlossenen Gefäß unter dem Eigendruck des Systems bewirkt, daß keine Maßnahmen getroffen werden müssen, um freiwerdendes Schwefeldioxid abzufangen. (Es findet keine $SO_2$-Emission statt). Es kann auch mit äquivalenten Mengen oder einem sehr geringen Überschuß an Sulfit eine befriedigend hohe Reaktionsgeschwindigkeit erzielt werden. Schließlich besitzt die Verwendung des bei der Addition entstehenden Produkts als Dispergator gegenüber der Verwendung anderer Dispergatoren den Vorteil, daß das entstehende Additionsprodukt nicht durch einen Dispergator anderer chemischer Struktur verunreinigt ist.

Zur Durchführung des erfindungsgemäßen Verfahrens stellt man zuerst eine wäßrige Dispersion her. Diese Dispersion enthält mindestens 4 Komponenten, nämlich

a) Maleinsäuredialkylester und/oder Fumarsäuredialkylester

b) ein Sulfit eines Alkalimetalls

c) das Sulfobernsteinsäuredialkylestersalz, welches durch Addition des Sulfits b) an den Diester a) entsteht, als Dispergator

d) ein Alkalimetallhydroxid.

Das Mengenverhältnis der Komponenten a) und b) zueinander wird so bemessen, daß pro C = C-Doppelbindung der Komponente a) 1,0 bis 1,1 Schwefelatome der Komponente b) vorliegen. Liegen pro C = C-Doppelbindung genau 1,0 Schwefelatome vor, handelt es sich um stöchiometrische Mengen an Sulfit. Liegen zwischen 1,0 und 1,1 Schwefelatome pro C = C-Doppelbindung vor, so ist ein bis zu 10 %iger Überschuß an Sulfit anwesend. Zweckmäßigerweise arbeitet man jedoch mit einem geringeren Überschuß, nämlich in der Größenordnung von nur 1 bis 2 %. Dies bedeutet, daß vorzugsweise pro C = C-Doppelbindung 1,01 bis 1,02 Schwefelatome anwesend sind. Die Menge an Komponente d) (Alkalimetallhydroxid), die bei der Herstellung der Dispersion zugefügt wird, beträgt 0,1 bis 4 Gew.%, bezogen auf Gesamtdispersion und auf 100 %iges Alkalimetallhydroxid.

Die Komponente c) ist dasjenige Sulfobernsteinsäuredialkylestersalz, das bei der Addition des Sulfits (Komponente b)) an den betreffenden Maleinsäuredialkylester bzw. Fumarsäuredialkylester (Komponente a)) entsteht. Dieses Salz wirkt wegen seiner oberflächenaktiven Eigenschaften als Dispergator und wird dem System von Anfang an in einer Menge von 1 bis 10 Gew.%, bezogen auf Gesamtdispersion, zugesetzt. Hierdurch wird eine homogene wäßrige Dispersion der Komponenten a), b), c) und d) erhalten und somit homogene Reaktionsführung ermöglicht. Die im Einzelfall zu verwendende Menge des Satzes (Komponente c)) ist demnach so zu bemessen, daß eine homogene Dispersion entsteht. Diese Menge hängt unter anderem von der Art des Diesters (Komponente a)) und von der gewünschten Konzentration der Dispersion ab. In einer Reihe von Fällen hat sich ein Zusatz von 3 bis 7 Gew.% an Komponente c), bezogen auf Gesamtdispersion, als vorteilhaft erwiesen.

Als Alkalimetallhydroxid (Komponente d)) verwendet man bevorzugt Natriumhydroxid in einer Menge von 0,5 bis 1,5 Gew.% oder Kaliumhydroxid in einer Menge von 0,9 bis 2,6 Gew.%, jeweils bezogen auf Gesamtdispersion.

Die Herstellung der wäßrigen Dispersion, welche die Komponenten a), b), c) und d) enthält, kann nach üblichen Methoden unter Zuhilfenahme gebräuchlicher apparativer Einrichtungen erfolgen. Die Reihenfolge der Zugabe der Komponenten a), b) c) und d) zu Wasser ist ebenfalls nicht kritisch. So kann z.B. der Diester (Komponente a)) in eine wäßrige Lösung des Sulfobernsteinsäurediestersalzes (Komponente c)) eingerührt werden, wobei das Sulfit (Komponente b)) entweder bereits in dieser Lösung enthalten ist oder nach Einrühren des Diesters in fester Form oder als wäßrige Lösung zugegeben wird.

Die Komponente a) (Maleinsäuredialkylester oder Fumarsäuredialkylester) ist ein Diester aus der betreffenden Säure und einem gesättigten aliphatischen Alkohol. Die beiden Esterfunktionen können aus dem gleichen oder aus verschiedenen Alkoholen gebildet sein. Die Komponente a) kann daneben auch aus einem Gemisch von Maleinsäuredialkylester und Fumarsäuredialkylester bestehen, wobei die zugrundeliegenden Alkohole gleicher oder verschiedener Natur sein können. In jedem Fall jedoch handelt es sich bei allen aus der Alkoholkomponente stammenden Alkylresten um gesättigte aliphatische Reste, welche linear oder verzweigt sein können. Diese Alkylreste können auch Substituenten tragen, vorausgesetzt, daß hierdurch die Addition des Alkalimetallsulfits an die C = C-Doppelbindung nicht behindert wird. Die Kettenlänge (Anzahl der C-Atome) der Alkylreste der Komponente a) ist nicht kritisch, jedoch muß gewährleistet sein, daß zusammen mit Sulfit (Komponente b)) und Dispergator (Komponente c)) eine homogene wäßrige Dispersion erhalten werden kann. Aus diesem Grund können sich Diester, deren Alkylgruppe eine gewisse Kettenlänge übersteigt, als ungeeignet erweisen. Bevorzugt verwendet man Diester eines linearen oder verzweigten einwertigen Alkohols mit 3 bis 12 C-Atomen. Als besonders geeignet für das erfindungsgemäße Verfahren hat sich der Diester aus Malein- oder Fumarsäure und 2-Ethyl-n-hexanol(1) erwiesen.

Die Maleinsäure- bzw. Fumarsäuredialkylester können nach bekannten Methoden, z.B. durch Umsetzung von Maleinsäureanhydrid, Maleinsäure, Fumarsäure oder deren Derivaten mit Alkoholen unter Säurekatalyse hergestellt werden.

Die Komponente b) der wäßrigen Dispersion, welche für das erfindungsgemäße Verfahren eingesetzt wird, ist ein Sulfit eines Alkalimetalls. Hierunter fallen Sulfite $M_2SO_3$, Hydrogensulfite $MHSO_3$ und auch Pyrosulfite der Formel $M_2S_2O_5$, wobei M für ein Alkalimetall steht. Als Alkalimetalle kommen in erster Linie Natrium oder Kalium in Frage. Besonders bevorzugt ist die Verwendung von Natriumpyrosulfit $Na_2S_2O_5$.

Als Dispergator (Komponente c) wird zur Herstellung der wäßrigen Dispersionen, welche für das erfindungsgemäße Verfahren eingesetzt werden, dasjenige Sulfobernsteinsäurediestersalz eingesetzt, welches bei der Addition des Sulfits (Komponente b)) an den Maleinsäure- bzw. Fumarsäuredialkylester entsteht bzw. es wird, wenn ein Salzgemisch entsteht, dieses Gemisch eingesetzt. Die Herstellung des als Dispergator (Komponente c)) verwendeten Sulfobernsteinsäurediestersalzes muß nicht nach dem erfindungsgemäßen Verfahren, sondern kann nach einem beliebigen bekannten Verfahren erfolgen. Ein bequemer Weg besteht jedoch darin, daß man einen entsprechenden Anteil des Additionsproduktes aus einem vorangegangenen Ansatz abzweigt.

Es hat sich als vorteilhaft erwiesen, wenn die wäßrige Dispersion außer den beschriebenen Komponenten a), b), c) und d) noch zusätzlich Methanol in einer Menge von 3 bis 5 Gew.% und/oder Ethylendiamintetraessigsäure in einer Menge von 0,05 bis 1 Gew.% und/oder 3 bis 7 Gew.% 2-Ethyl-n-hexanol (1) enthält. Diese Mengenangaben sind jeweils auf Gesamtdispersion bezogen. Da eine Erhöhung der Konzentration der Reaktionsteilnehmer zu einer Erhöhung der Reaktionsgeschwindigkeit führt, ist es zweckmäßig, in nicht zu verdünnter Dispersion zu arbeiten. Durch die Forderung nach Stabilität der homogenen wäßrigen Dispersion sind der Konzentration jedoch nach oben Grenzen gesetzt. Vorzugsweise wendet man daher für das erfindungsgemäße Verfahren Dispersionen an, welche zu 20 bis 40 Gew.% aus Wasser und zu 80 bis 60 Gew.% aus den übrigen Komponenten bestehen.

Eine günstige Methode zur Durchführung des erfindungsgemäßen Verfahrens besteht darin, folgendermaßen vorzugehen: Man legt eine wäßrige Lösung von Sulfit, z.B. $Na_2S_2O_5$ vor, der ggf. Ethylendiamintetraessigsäure zugefügt ist. Anschließend werden Malein- bzw. Fumarsäurediester, Sulfobernsteinsäurediestersalz (Komponente c)) und Alkalimetallhydroxid zugegeben. Das anschließende Aufheizen kann bei Umgebungsdruck erfolgen, bis eine Temperatur zwischen 95 und 100 °C erreicht ist. Nach dem gasdichten Verschließen der Apparatur wird auf die gewünschte Temperatur zwischen 120 und 150 °C geheizt. Soll mit einem Zusatz von Methanol, wie oben beschrieben, gearbeitet werden, wird Methanol zweckmäßigerweise nach Schließen der Apparatur zugegeben.

Zur Durchführung der Additionsreaktion wird die wäßrige Dispersion so lange erhitzt, bis 95 bis 100 %, vorzugsweise 100% der Doppelbindungen des Maleinsäure- bzw. Fumarsäuredialkylesters umgesetzt sind, d.h. in $-CH_2-CH(SO_3M)$-Gruppierungen umgewandelt sind, wobei M für ein Alkalimetall steht. Der Zeitpunkt, zu dem dies der Fall ist, hängt u.a. von Art und Konzentration der Ausgangsverbindungen ab und läßt sich durch entsprechende analytische Bestimmungen ermitteln. Normalerweise wird für die Umsetzung eine Zeit von 15 bis 60 Minuten benötigt. Besonders kurze Reaktionszeiten lassen sich erreichen, wenn der Dispersion 0,5 bis 1,5 Gew.% Natriumhydroxid zugefügt wurden. Das Erhitzen der Dispersion wird in einem geschlossenen, d.h. auch bei erhöhtem Druck gasdichten, Gefäß bei einer Temperatur im Bereich von 120 bis 150 °C, vorzugsweise 125 - 135 °C, durchgeführt. Die Verwendung eines gasdichten Gefäßes führt dazu, daß die Reaktion unter dem Eigendruck des Systems abläuft, der bei der betreffenden Temperatur vorliegt.

Als geschlossene Gefäße für die Umsetzung können bekannte Druckgefäße, wie Autoklaven, verwendet werden. Nach Beendigung der Umsetzung kann die erhaltene wäßrige Lösung oder Dispersion von Sulfobernsteinsäuredialkylestersalz anschließend, wenn gewünscht, in üblicher Weise zur Isolierung und Reinigung des Sulfobernsteinsäuredialkylestersalzes aufgearbeitet werden.

Das erfindungsgemäße Verfahren wird nunmehr an Hand eines Ausführungsbeispiels veranschaulicht.

Beispiel

In 242 l Wasser, das 1 kg Ethylendiamintetraessigsäure enthielt, wurden 137 kg Natriumbisulfit ($Na_2S_2O_5$) bei Raumtemperatur gelöst. Anschließend wurden 484 kg Maleinsäure-Di-[2-ethyl-n-hexyl(1)]ester, 50 kg Natriumsalz des Sulfobernsteinsäuredi-(2-ethyl-n-hexyl(1)-esters, 55 kg 2-Ethyl-n-hexanol(1) und 2kg 50 %iger wäßriger Natriumhydroxidlösung unter Rühren zugegeben. In einem gasdicht verschließbaren Kessel wurde die erhaltene Dispersion bei geöffnetem Kessel auf 96 °C geheizt. Sobald die Dispersion eine Temperatur von 96 °C erreicht hatte, wurde der Kessel geschlossen und es wurden 30 kg Methanol eingeleitet. Es wurde weiter bis zum Erreichen einer Innentemperatur von 120 °C geheizt und das Gemisch bei 120 - 130 °C gehalten, bis das Reaktionsgemisch wasserklar war. Nach etwa 20 Minuten wurde auf 30 °C abgekühlt. Eine der Apparatur entnommene Probe der Lösung war klar und besaß einen pH-Wert von etwa 6,3.

**Patentansprüche**

1. Verfahren zur Herstellung von Sulfobernsteinsäuredialkylestersalzen in wäßriger Dispersion oder Lösung durch Erhitzen einer wäßrigen Dispersion, welche

   a) Maleinsäuredialkylester und/oder Fumarsäuredialkylester

   b) ein Sulfit eines Alkalimetalls und

   c) das Sulfobernsteinsäuredialkylestersalz, welches durch Addition des Sulfits b) an den Diester a) entsteht, als Dispergator

   enthält, in der die Komponenten a) und b) in solchen Mengen enthalten sind, daß pro C = C-Doppelbindung der Komponente a) 1,0 bis 1,1 Schwefelatome der Komponente b) vorliegen, und in der die Komponente c) in einer Menge von 1 bis 10 Gew.%, bezogen auf Gesamtdispersion, enthalten ist, wobei man diese Dispersion so lange erhitzt, bis 95 bis 100 % der C = C-Doppelbindungen umgesetzt sind, dadurch gekennzeichnet, daß das Erhitzen der Dispersion in einem geschlossenen Gefäß unter dem Eigendruck des Systems bei einer Temperatur im Bereich von 120 bis 150°C durchgeführt wird und daß der Dispersion vor dem Erhitzen ein Alkalimetallhydroxid in einer Menge von 0,1 bis 4 Gew.%, bezogen auf Gesamtdispersion, hinzugefügt wird.

2. Verfahren nach Patentanspruch 1, dadurch gekennzeichnet, daß als Komponente b) $Na_2S_2O_5$ eingesetzt wird.

3. Verfahren nach Patentanspruch 1 oder 2, dadurch gekennzeichnet, daß als Komponente a) ein Diester eines linearen oder verzweigten einwertigen Alkohols mit 3 bis 12 C-Atomen eingesetzt wird.

4. Verfahren nach Patentanspruch 3, dadurch gekennzeichnet, daß der Diester von 2-Ethyl-n-hexanol(1) eingesetzt wird.

5. Verfahren nach einem oder mehreren der Patentansprüche 1 bis 4, dadurch gekennzeichnet, daß die Komponenten a) und b) in solchen Mengen eingesetzt werden, daß pro C = C-Doppelbindung der Komponente a) 1,01 bis 1,02 Schwefelatome der Komponente b) vorliegen.

6. Verfahren nach einem oder mehreren der Patentansprüche 1 bis 5, dadurch gekennzeichnet, daß der Dispersion als Alkalimetallhydroxid Natriumhydroxid in einer Menge von 0,5 bis 1,5 Gew.% oder Kaliumhydroxid in einer Menge von 0,9 bis 2,6 Gew.%, bezogen auf Gesamtdispersion, vor dem Erhitzen hinzugefügt werden.

7. Verfahren nach einem oder mehreren der Patentansprüche 1 bis 6, dadurch gekennzeichnet, daß die Dispersion 3 bis 5 Gew.% Methanol und/oder 0,05 bis 1 Gew.% Ethylendiamintetraessigsäure und/oder 3 bis 7 Gew.% 2-Ethyl-n-hexanol(1), jeweils bezogen auf Gesamtdispersion, enthält.